# EUROPEAN PATENT APPLICATION

(11) **EP 4 130 165 A1**
(43) Date of publication of application: **08.02.2023**
(21) Application number: 21852929.5
(22) Date of filing: 02.08.2021
(51) Int. Cl.: C09C 3/08, A61Q 1/02, A61K 8/19, A61K 8/25, A61K 8/27, A61K 8/29, A61K 8/39

(54) **POWDER MATERIAL FOR COSMETIC, METHOD FOR PRODUCING POWDER MATERIAL FOR COSMETIC, AND COSMETIC**

(30) Priority: 07.08.2020 JP 2020134477
(71) Applicant: Daito Kasei Kogyo Co., Ltd., Osaka-shi, Osaka 535-0005 (JP)
(72) Inventor: TSUCHIYA Reiichiro, Osaka-shi, Osaka 535-0005 (JP); HATTORI Haruka, Osaka-shi, Osaka 535-0005 (JP); ODA Yayoi, Osaka-shi, Osaka 535-0005 (JP)
(74) Representative: Cabinet Beau de Loménie
(86) International application number: PCT/JP2021/028668
(87) International publication number: WO 2022/030462

(57) **Abstract**

Provided is a powder material for a cosmetic that has excellent dispersibility in any of silicone oil agents, hydrocarbon oil agents, and ester oil agents, and does not affect the color appearance of a formulation. The powder material for a cosmetic contains a powder for a cosmetic surface-treated with an ester compound of a fatty acid having 8-20 carbon atoms and glycerol. The ester compound is at least one selected from the group consisting of diglyceryl tetraisostearate, diglyceryl triisostearate, glyceryl tri(caprylate/caprate), glyceryl dilaurate, glyceryl triisostearate, decaglyceryl monoisostearate, and decaglyceryl diisostearate.

## Description

### TECHNICAL FIELD

The present invention relates to a powder material for a cosmetic that has excellent dispersibility in an oil agent, a method for producing the powder material for a cosmetic, and a cosmetic containing the powder material for a cosmetic.

### BACKGROUND ART

The dispersibility of powder in an oil agent is a key factor that determines properties of a formulation such as appearance, function, stability, and feeling of use. In order to improve dispersibility in an oil agent, the surface of a powder material for a cosmetic may conventionally be coated with isopropyltriisostearoyl titanate and thereby lipophilized. The resultant powder material may be used in foundation and the like. Isopropyltriisostearoyl titanate has, in the molecule, a plurality of isostearyl groups, which have a branched structure. The powder material for a cosmetic that is surface-treated with isopropyltriisostearoyl titanate, which has such a molecular structure, has higher lipophilicity, and therefore, has excellent dispersibility in any of silicone oil agents, hydrocarbon oil agents, and ester oil agents that are used in cosmetic formulations.

However, isopropyltriisostearoyl titanate itself has a red color, and therefore, a powder material for a cosmetic that is surface-treated with isopropyltriisostearoyl titanate also has a red color, which disadvantageously affects the color appearance of a formulation. Under these circumstances, other surface treatment agents such as a polyhydroxy fatty acid or a derivative thereof (see, for example, Patent Document 1) and a saturated fatty acid triglyceride (see, for example, Patent Document 2) have been studied as a substitute for isopropyltriisostearoyl titanate.

### CITATION LIST

### PATENT LITERATURE

Patent Document 1: Japanese Unexamined Patent Application Publication No. 2006-291199
Patent Document 2: Japanese Unexamined Patent Application Publication No. S61-176667

### SUMMARY OF INVENTION

### TECHNICAL PROBLEM

However, the surface-treatment pigment material of Patent Document 1 has good dispersibility in hydrocarbon oil agents and ester oil agents, but poor dispersibility in silicone oil agents. The extender pigment of Patent Document 2, in which a saturated fatty acid triglyceride, which is used as a surface treatment agent, does not have a branched structure at an alkyl chain in the molecule, has much lower dispersibility in any of silicone oil agents, hydrocarbon oil agents, and ester oil agents than that of a powder material for a cosmetic that is surface-treated with isopropyltriisostearoyl titanate.

With the above problem in mind, the present invention has been made. It is an object of the present invention to provide: a powder material for a cosmetic that has excellent dispersibility in any of silicone oil agents, hydrocarbon oil agents, and ester oil agents, and does not affect the color appearance of a formulation; a method for producing the powder material for a cosmetic; and a cosmetic containing the powder material for a cosmetic.

### SOLUTION TO PROBLEM

To attain the above object, a powder material for a cosmetic according to the present invention comprises:
a powder for a cosmetic surface-treated with an ester compound of a fatty acid having 8-20 carbon atoms and glycerol.

In the powder material for a cosmetic having the above feature, the powder for a cosmetic is surface-treated with the ester compound of a fatty acid having 8-20 carbon atoms and glycerol. Therefore, the powder material for a cosmetic has excellent dispersibility in any of silicone oil agents, hydrocarbon oil agents, and ester oil agents. In addition, the ester compound of a fatty acid having 8-20 carbon atoms and glycerol is colorless, and therefore, the powder for a cosmetic surface-treated with the ester compound is not colored, and is not likely to affect the color appearance of a formulation.

In the powder material for a cosmetic of the present invention,
the ester compound is preferably at least one selected from the group consisting of diglyceryl tetraisostearate, diglyceryl triisostearate, glyceryl tri(caprylate/caprate), glyceryl dilaurate, glyceryl triisostearate, decaglyceryl monoisostearate, and decaglyceryl diisostearate.

In the powder material for a cosmetic having the above feature, the ester compound is at least one selected from the group consisting of diglyceryl tetraisostearate, diglyceryl triisostearate, glyceryl tri(caprylate/caprate), glyceryl dilaurate, glyceryl triisostearate, decaglyceryl monoisostearate, and decaglyceryl diisostearate, which have, in the molecule, at least one isostearyl group, which has a branched structure, or at least one lauryl group, caprylic group, or capric group, which has a straight-chain structure, and have a monoglyceryl group, diglyceryl group, or decaglyceryl group. Therefore, the powder for a cosmetic has higher lipophilicity, and therefore, has more excellent dispersibility in silicone oil agents, hydrocarbon oil agents, and ester oil agents.

In the powder material for a cosmetic of the present invention,
the contained amount of the ester compound is preferably 0.1-10 mass%.

In the powder material for a cosmetic having the above feature, the contained amount of the ester compound is 0.1-10 mass%, and therefore, a dispersion is not formed in which the ester compound is present between particles of the powder for a cosmetic, and the ester compound covers the surface of the powder for a cosmetic, so that the powder material for a cosmetic has powder properties as a whole. As a result, the powder for a cosmetic has higher lipophilicity, and therefore, has more excellent dispersibility in silicone oil agents, hydrocarbon oil agents, and ester oil agents.

In the powder material for a cosmetic of the present invention,
the powder for a cosmetic is preferably at least one selected from the group consisting of titanium oxide, yellow iron oxide, red iron oxide, black iron oxide, talc, zinc oxide, silicon oxide, pearl mica, mica, and sericite.

In the powder material for a cosmetic having the above feature, if the powder for a cosmetic is selected from the above appropriate ones, the function, stability, feeling of use, and the like of make-up cosmetics such as foundation, eye shadow, eyebrow liner, and blush to which these powders for a cosmetic are added as pigment can be improved without the color appearance of a formulation being affected by the color of the ester compound.

To attain the above object, a method for producing a powder material for a cosmetic according to the present invention, comprises:
mixing a powder for a cosmetic with a liquid mixture obtained by dispersing, suspending, or dissolving an ester compound of a fatty acid having 8-20 carbon atoms and glycerol in an organic solvent; and
heat-treating the mixture obtained in the mixing.

In the method having the above feature, which comprises mixing a powder for a cosmetic with a liquid mixture obtained by dispersing, suspending, or dissolving an ester compound of a fatty acid having 8-20 carbon atoms and glycerol in an organic solvent, and heat-treating the mixture obtained in the mixing, a powder material for a cosmetic containing the powder for a cosmetic surface-treated with the ester compound of a fatty acid having 8-20 carbon atoms and glycerol can be obtained. The powder material for a cosmetic, in which the powder for a cosmetic is surface-treated with the ester compound of a fatty acid having 8-20 carbon atoms and glycerol, has excellent dispersibility in any of silicone oil agents, hydrocarbon oil agents, and ester oil agents. In addition, the ester compound of a fatty acid having 8-20 carbon atoms and glycerol is colorless, and therefore, the powder for a cosmetic surface-treated with the ester compound is not colored, and is not likely to affect the color appearance of a formulation.

In the method for producing a powder material for a cosmetic of the present invention,
in the heat-treating, a treatment temperature is preferably set to 70-150°C.

In the method having the above feature, the treatment temperature in the heat-treating is set to 70-150°C. As a result, the ester compound of a fatty acid having 8-20 carbon atoms and glycerol is present on the surface of the powder for a cosmetic with the hydrophilic group of the ester compound pointing toward the powder for a cosmetic, resulting in an increase in the lipophilicity of the powder for a cosmetic. This can provide a powder material for a cosmetic having more excellent dispersibility in silicone oil agents, hydrocarbon oil agents, and ester oil agents.

In the method for producing a powder material for a cosmetic of the present invention,
in the mixing, the liquid mixture is preferably added to the powder for a cosmetic while the powder for a cosmetic is being stirred.

In the method having the above feature, in the mixing, the liquid mixture is added to the powder for a cosmetic while the powder for a cosmetic is being stirred. Therefore, the surface of the powder for a cosmetic can be quickly uniformly coated with a small amount of the ester compound, which is a surface treatment agent.

To attain the above object, a cosmetic according to the present invention comprises the above powder material for a cosmetic.

The cosmetic having the above feature, which contains the above powder material for a cosmetic, has excellent function, stability, and feeling of use. In addition, the ester compound used in the surface treatment is colorless, and therefore, the powder for a cosmetic surface-treated with the ester compound is not colored, and is not likely to affect the color appearance of a formulation.

### BRIEF DESCRIPTION OF DRAWINGS

[FIG. 1] FIG. 1 is a graph showing the result of an oil agent dispersibility assessment test 1.
[FIG. 2] FIG. 2 is a torque curve obtained by determination of an oil absorption number using an ester oil agent.
[FIG. 3] FIG. 3 is a torque curve obtained by determination of an oil absorption number using a hydrocarbon oil agent.
[FIG. 4] FIG. 4 is a torque curve obtained by determination of an oil absorption number using a silicone oil agent.
[FIG. 5] FIG. 5 is a graph showing the result of an oil agent dispersibility assessment test 2.
[FIG. 6] FIG. 6 is a graph showing the result of a water repellency test.

### DESCRIPTION OF EMBODIMENTS

A powder material for a cosmetic, a method for producing a powder material for a cosmetic, and a cosmetic according to the present invention will be described in detail below. Note that the present invention is not intended to be limited to embodiments and examples described below.

### [Powder Material for Cosmetic]

The powder material for a cosmetic of the present invention has excellent dispersibility in any of silicone oil agents, hydrocarbon oil agents, and ester oil agents, and contains a powder for a cosmetic that is surface-treated with an ester compound of a fatty acid having 8-20 carbon atoms and glycerol.

### <Ester Compound>

In the powder material for a cosmetic of the present invention, the ester compound of a fatty acid having 8-20 carbon atoms and glycerol is used as a surface treatment agent for lipophilizing the powder for a cosmetic. The ester compound of a fatty acid having 8-20 carbon atoms and glycerol is colorless, and therefore, is not likely to affect the color of the powder for a cosmetic even when the powder for a cosmetic is surface-treated with the ester compound. Of the fatty acids, fatty acids having 8-18 carbon atoms are preferable. The fatty acid is either branched or straight. Glycerol includes monoglycerol, which is not a polymer (i.e., a single molecule), and polyglycerol, which is a polymer (i.e., glycerol molecules are polymerized). In the case where polyglycerol is used, the degree of polymerization thereof is not particularly limited, and is preferably 2 to 10. Glycerol is preferably monoglycerol, which is a single molecule, diglycerol, whose degree of polymerization is 2, or decaglycerol, whose degree of polymerization is 10. Examples of the ester compound of a fatty acid having 8-20 carbon atoms and glycerol include diglyceryl tetraisostearate (DG4ISA), diglyceryl triisostearate (DG3ISA), diglyceryl diisostearate, diglyceryl monoisostearate, glyceryl tri(caprylate/caprate), glyceryl triethylhexanoate, glyceryl dilaurate, glyceryl triisostearate, decaglyceryl monoisostearate, and decaglyceryl diisostearate. Of them, DG4ISA, DG3ISA, glyceryl dilaurate, glyceryl triisostearate, decaglyceryl monoisostearate, and decaglyceryl diisostearate are preferable. DG4ISA, DG3ISA, glyceryl tri(caprylate/caprate), glyceryl dilaurate, glyceryl triisostearate, decaglyceryl monoisostearate, and decaglyceryl diisostearate have, in the molecule, at least one of an isostearyl group, which has a branched structure, or a lauryl group, caprylic group, or capric group, which has a straight-chain structure, and have a monoglyceryl group, diglyceryl group, or decaglyceryl group, and therefore, can impart particularly excellent lipophilicity to the powder for a cosmetic when used to surface-treat the powder. In addition, DG4ISA, DG3ISA, glyceryl tri(caprylate/caprate), glyceryl dilaurate, glyceryl triisostearate, decaglyceryl monoisostearate, and decaglyceryl diisostearate are a naturally-occurring material, and therefore, can be used, without a worry, by health-conscious cosmetic users who do not like chemically synthesized products. Note that the above ester compounds may be used in combination.

The contained amount of the ester compound in the powder material for a cosmetic of the present invention is preferably 0.1-10 mass%, more preferably 0.5-10 mass%. If the contained amount of the ester compound is within the above range, a dispersion is not formed in which the ester compound is present between particles of the powder for a cosmetic, and the ester compound covers the surface of the powder for a cosmetic, so that the powder material for a cosmetic has powder properties as a whole. As a result, the powder for a cosmetic has higher lipophilicity, and therefore, has more excellent dispersibility in silicone oil agents, hydrocarbon oil agents, and ester oil agents. In addition, when the powder material for a cosmetic is dispersed in a silicone oil agent, a hydrocarbon oil agent, or an ester oil agent, the resultant oil agent has an appropriate viscosity, resulting in a cosmetic having excellent feeling of use. Furthermore, the contained amount of the ester compound is not greater than necessary, resulting in a reduction in manufacturing cost of a cosmetic. Because the contained amount of the ester compound is at least 0.1 mass%, the powder for a cosmetic can be sufficiently covered, and therefore, sufficient lipophilicity can be imparted to the powder for a cosmetic. In addition, when the powder for a cosmetic is dispersed in a silicone oil agent, a hydrocarbon oil agent, or an ester oil agent, the viscosity of the oil agent can be reduced. Because the contained amount of the ester compound is at most 10 mass%, the contained amount of the ester compound is not greater than necessary, which is beneficial in terms of cost. In addition, it is possible to inhibit an excessive amount of the ester compound from being present between particles of the powder for a cosmetic, and therefore, the powder material for a cosmetic is inhibited from having dispersion properties as a whole, and appropriate lipophilicity can be imparted to the powder for a cosmetic. Here, if after the powder for a cosmetic is surface-treated with the ester compound, the powder material for a cosmetic aggregates in a powder state, it is likely to be difficult for the powder material for a cosmetic to be used in particularly powder cosmetics (powder foundation, eye shadow, blush, etc.) in terms of texture, processing (the powder material for a cosmetic cannot be mixed with other colors), or the like. In this regard, because the contained amount of the ester compound is at most 10 mass%, the powder material for a cosmetic can be inhibited from aggregating in a powder state, which advantageously does not set a limitation on use in the above powder cosmetics.

### <Powder for Cosmetic>

The powder for a cosmetic is a base for the powder material for a cosmetic of the present invention. Examples of the powder for a cosmetic include: inorganic pigments such as titanium oxide, yellow iron oxide, red iron oxide, black iron oxide, talc, zinc oxide, silicon oxide, pearl mica, mica, carbon black, iron red, cerium oxide, manganese violet, cobalt violet, chromium oxide, chromium hydroxide, cobalt titanate, ultramarine blue, iron blue, magnesium oxide, zirconium oxide, kaolinite, sericite, muscovite, phlogopite, synthetic mica, lepidolite, biotite, vermiculite, silica, calcium carbonate, magnesium carbonate, magnesium silicate, aluminum silicate, barium silicate, calcium silicate, barium sulfate, calcium sulfate, calcium phosphate, hydroxyapatite, boron nitride, pearl pigment, and bismuth oxychloride; organic colored elements such as Red No. 3, Red No. 10, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Yellow No. 4, Yellow No. 5, Yellow No. 202, Yellow No. 203, Yellow No. 205, Yellow No. 401, Orange No. 201, Orange No. 203, Orange No. 204, Orange No. 205, Orange No. 206, Orange No. 207, Blue No. 1, Blue No. 2, Blue No. 201, Blue No. 404, Green No. 3, Green No. 201, Green No. 204, and Green No. 205; natural colored elements such as chloropyll and β-carotene; metallic soaps such as zinc myristate, calcium palmitate, and aluminum stearate; and organic powders such as tar pigment, nylon powder, cellulose powder, polyethylene powder, polymethyl methacrylate powder, polystyrene powder, acrylic powder, and silicone powder. These powders for a cosmetic can be used alone or in combination. In particular, the powder material for a cosmetic of the present invention contains a colorless ester compound of a fatty acid having 8-20 carbon atoms and glycerol as a surface treatment agent, and therefore, does not affect the color of the powder for a cosmetic surface-treated with the ester compound or the color appearance of a formulation. Therefore, the powder material for a cosmetic of the present invention can be suitably used in pigment powders that are added to make-up cosmetics such as foundation, eye shadow, eyebrow liner, and blush, e.g., titanium oxide, yellow iron oxide, red iron oxide, black iron oxide, talc, zinc oxide, silicon oxide, pearl mica, mica, and sericite.

### <Other Components>

The powder material for a cosmetic of the present invention can contain other components in addition to the ester compound and the powder for a cosmetic. Examples of such components include silicones, alkylsilanes, alkyltitanates, fluorine compounds, amino acid compounds, and fatty acid compounds. These components can be used in combination with the ester compound in the surface treatment of the powder for a cosmetic.

### [Method for Producing Powder Material for Cosmetic]

The powder material for a cosmetic of the present invention can be produced by carrying out steps (I) and (II) below.

### (I) Mixing Step

In a mixing step, an ester compound of a fatty acid having 8-20 carbon atoms and glycerol is dispersed, suspended, or dissolved in an organic solvent to prepare a liquid mixture, and the liquid mixture is mixed with a powder for a cosmetic. Examples of the organic solvent include hydrocarbon solvents, alcoholic solvents, and high-polarity organic solvents (acetone and ethyl acetate). One of these organic solvents in which a specific ester compound can be appropriately dispersed, suspended, or dissolved can be used. For example, in the case where DG4ISA is used as the ester compound, a hydrocarbon solvent having low polarity such as hexane is preferably used as the organic solvent. In the case where DG3ISA, diglyceryl diisostearate, diglyceryl monoisostearate, glyceryl dilaurate, glyceryl triisostearate, decaglyceryl monoisostearate, or decaglyceryl diisostearate is used as the ester compound, any of hydrocarbon solvents, alcoholic solvents, and high-polarity organic solvents can be suitably used as the organic solvent. By previously dispersing, suspending, or dissolving the ester compound in the organic solvent, the surface of the powder for a cosmetic can be easily uniformly coated with a small amount of the ester compound, which is a surface treatment agent. The amount of the organic solvent is preferably 10-30 mass% of the powder for a cosmetic which is mixed with the liquid mixture. If the amount of the organic solvent is within the above range, then when the contained amount of the ester compound in the powder material for a cosmetic is set to 0.1-10 mass%, the surface of the powder for a cosmetic is more easily uniformly coated with the ester compound. Because the amount of the organic solvent is at least 10 mass% of the powder for a cosmetic, the surface of the powder for a cosmetic can be uniformly coated with the ester compound even if the wettability of the powder for a cosmetic is low with respect to the organic solvent. As a result, the time it takes to mix the powder for a cosmetic with the liquid mixture can be reduced, resulting in an improvement in the manufacturing efficiency of the powder material for a cosmetic. Because the amount of the organic solvent is at most 30 mass% of the powder for a cosmetic, the time it takes to remove the organic solvent after mixing of the liquid mixture with the powder for a cosmetic can be reduced, resulting in an improvement in the manufacturing efficiency of the powder material for a cosmetic. The liquid mixture and the powder for a cosmetic may be mixed together using a mixer, such as a Henschel mixer, loedige mixer, kneader, V-type mixer, or roll mill. When a mixer is used, the liquid mixture is preferably gradually added to and mixed with the powder for a cosmetic while the powder for a cosmetic is being stirred. In addition, in the production method of present invention, n-hexane, isopropyl alcohol, or the like, which has a boiling point lower than that of the ester compound of a fatty acid having 8-20 carbon atoms and glycerol, is preferably used as the organic solvent, and the organic solvent is preferably vaporized and removed during or after mixing.

### (II) Heat Treatment Step

In a heat treatment step, the mixture obtained in the mixing step is subjected to a heat treatment. By the heat treatment, obtained is the powder material for a cosmetic of the present invention in which the surface of the powder for a cosmetic is coated with the ester compound of a fatty acid having 8-20 carbon atoms and glycerol. The treatment temperature in the heat treatment step is preferably set to 70-150°C. If the treatment temperature is within the above range, the ester compound of a fatty acid having 8-20 carbon atoms and glycerol is present on the surface of the powder for a cosmetic with the hydrophilic group of the ester compound pointing toward the powder for a cosmetic, resulting in an increase in the lipophilicity of the powder for a cosmetic in the powder material for a cosmetic of the present invention. This further improves dispersibility in silicone oil agents, hydrocarbon oil agents, or ester oil agents. Because the treatment temperature is 70°C or higher, the ester compound can be appropriately oriented, which imparts sufficient lipophilicity to the powder for a cosmetic. Because the treatment temperature is 150°C or lower, the ester compound can be inhibited from being partially vaporized or degraded, and therefore, the surface of the powder for a cosmetic can be appropriately coated with the ester compound, which can impart sufficient lipophilicity to the powder for a cosmetic. The treatment time in the heat treatment step is preferably set to 3-9 hours. If the treatment time is within the above range, the ester compound of a fatty acid having 8-20 carbon atoms and glycerol is oriented to an appropriate extent on the surface of the powder for a cosmetic. Because the treatment time is at least 3 hours, the ester compound is appropriately oriented, which can improve sufficient lipophilicity to the powder for a cosmetic. Because the treatment time is at most 9 hours, the ester compound can be inhibited from being partially vaporized or degraded, and therefore, the surface of the powder for a cosmetic can be appropriately coated with the ester compound, which can impart sufficient lipophilicity to the powder for a cosmetic. After the heat treatment, a pulverization treatment is preferably carried out. When pulverization is carried out after the heat treatment, a commonly used pulverizer, such as a hammer mill, ball mill, sand mill, or jet mill, can be used. Every pulverlizer can provide similar quality, and therefore, the pulverizer is not particularly limited.

It is preferable that the powder material for a cosmetic of the present invention should have sufficient lipophilicity. The degree of lipophilicity is represented by how much difficult to wet with water. The difficulty in wetting with water is represented by, for example, water repellency, which is represented by a contact angle for water. Specifically, the higher the lipophilicity, the higher the water repellency, and the greater the contact angle. Therefore, the lipophilicity of the powder material for a cosmetic of the present invention increases with an increase in the contact angle. Therefore, the contact angle of the powder for a cosmetic of the present invention is preferably at least 60°, more preferably at least 100°. While it is not particularly necessary to set an upper limit on the contact angle, about 150° is the limit below which a water droplet can maintain its spherical shape.

### [Cosmetic]

A cosmetic according to the present invention contains the above powder material for a cosmetic. The contained amount of the powder material for a cosmetic is not particularly limited, and is preferably 0.1-95 wt%. The above powder material for a cosmetic contained in the cosmetic allows the cosmetic to have excellent function, stability, and feeling of use due to the excellent dispersibility of the powder material for a cosmetic in the case where the cosmetic employs any of silicone oil agents, hydrocarbon oil agents, and ester oil agent. In addition, the ester compound used to surface-treat the powder material for a cosmetic is colorless, and therefore, the color appearance of the cosmetic containing the powder material for a cosmetic is not likely to be affected by the color of the ester compound.

The cosmetic of the present invention may further contain components that are commonly used in cosmetics, such as powders, surfactants, oil agents, gelling agents, polymers, beauty components, moisturizing agents, colored elements, preservatives, and fragrances in amounts that do not impair the effect of the present invention.

### [Examples]

### <Powder Material for Cosmetic>

Powder materials for a cosmetic according to the present invention (Examples 1 and 2) were produced and subjected to an oil agent dispersibility assessment test. The dispersion properties in an oil agent of the powder material for a cosmetic of Example 1 was also assessed. For comparison, powder materials for a cosmetic (Comparative Examples 1 and 2) that depart from the range of the present invention were produced and subjected to a similar test and assessment. In addition, powder materials for a cosmetic according to Examples 3-21 and Comparative Examples 3-7 were produced and subjected to tests or assessment described below.

### [Example 1]

Two grams of DG4ISA, which was to serve as a surface treatment agent, was added to and dispersed in 10 g of n-hexane to prepare a liquid mixture. Next, 98 g of titanium oxide as a powder for a cosmetic was stirred using a mixer. The liquid mixture was dropped to and mixed with the powder for a cosmetic for 10 minutes to prepare a mixture. The mixture was subjected to a heat treatment at 110°C for 6 hours, and the resultant dry powder was pulverized using a hammer mill, to obtain a powder material for a cosmetic according to Example 1. The contained amount of DG4ISA in the powder material for a cosmetic of Example 1 was 2.0 mass%.

### [Example 2]

A powder material for a cosmetic according to Example 2 was obtained in a manner similar to that of Example 1, except that DG3ISA was used as a surface treatment agent. The contained amount of DG3ISA in the powder material for a cosmetic of Example 2 was 2.0 mass%.

### [Example 3]

A powder material for a cosmetic according to Example 3 was obtained in a manner similar to that of Example 1, except that iron oxide (a mixture of yellow, red, and black iron oxides) was used as a powder for a cosmetic. The powder material for a cosmetic of Example 3 was added to a water-in-oil (W/O) type liquid foundation described below, and was used only for assessing the stability of the cosmetic.

### [Example 4]

A powder material for a cosmetic according to Example 4 was obtained in a manner similar to that of Example 1, except that talc powder was used as a powder for a cosmetic. The powder material for a cosmetic of Example 4 was added to a W/O type liquid foundation described below, and was used only for assessing the stability of the cosmetic.

### [Example 5]

A powder material for a cosmetic according to Example 5 was obtained in a manner similar to that of Example 2, except that iron oxide (a mixture of yellow, red, and black iron oxides) was used as a powder for a cosmetic. The powder material for a cosmetic of Example 5 was added to a W/O type liquid foundation described below, and was used only for assessing the stability of the cosmetic.

### [Example 6]

A powder material for a cosmetic according to Example 6 was obtained in a manner similar to that of Example 2, except that talc powder was used as a powder for a cosmetic. The powder material for a cosmetic of Example 6 was added to a W/O type liquid foundation described below, and was used only for assessing the stability of the cosmetic.

### [Example 7]

Two grams of glyceryl tri(caprylate/caprate), which was to serve as a surface treatment agent, was added to and dispersed in 10 g of isopropyl alcohol to prepare a liquid mixture. Next, 98 g of titanium oxide as a powder for a cosmetic was stirred using a mixer. The liquid mixture was dropped to and mixed with the powder for 10 minutes to prepare a mixture. The mixture was subjected to a heat treatment at 110°C for 6 hours, and the resultant dry powder was pulverized using a hammer mill, to obtain a powder material for a cosmetic according to Example 7. The contained amount of glyceryl tri(caprylate/caprate) in the powder material for a cosmetic of Example 7 was 2.0 mass%.

### [Example 8]

A powder material for a cosmetic according to Example 8 was obtained in a manner similar to that of Example 7, except that glyceryl triethylhexanoate was used as a surface treatment agent. The contained amount of glyceryl triethylhexanoate in the powder material for a cosmetic of Example 8 was 2.0 mass%.

### [Example 9]

A powder material for a cosmetic according to Example 9 was obtained in a manner similar to that of Example 7, except that glyceryl dilaurate was used as a surface treatment agent. The contained amount of glyceryl dilaurate in the powder material for a cosmetic of Example 9 was 2.0 mass%.

### [Example 10]

A powder material for a cosmetic according to Example 10 was obtained in a manner similar to that of Example 7, except that glyceryl triisostearate was used as a surface treatment agent. The contained amount of glyceryl dilaurate in the powder material for a cosmetic of Example 10 was 2.0 mass%.

### [Example 11]

A powder material for a cosmetic according to Example 11 was obtained in a manner similar to that of Example 7, except that decaglyceryl monoisostearate was used as a surface treatment agent. The contained amount of decaglyceryl monoisostearate in the powder material for a cosmetic of Example 11 was 2.0 mass%.

### [Example 12]

A powder material for a cosmetic according to Example 12 was obtained in a manner similar to that of Example 7, except that decaglyceryl diisostearate was used as a surface treatment agent. The contained amount of decaglyceryl diisostearate in the powder material for a cosmetic of Example 12 was 2.0 mass%.

### [Example 13]

One-tenth of a gram of DG4ISA, which was to serve as a surface treatment agent, was added to and dispersed in 10 g of n-hexane to prepare a liquid mixture. Next, 99.9 g of titanium oxide as a powder for a cosmetic was stirred using a mixer. The liquid mixture was dropped to and mixed with the powder for a cosmetic for 10 minutes to prepare a mixture. The mixture was subjected to a heat treatment at 110°C for 6 hours, and the resultant dry powder was pulverized using a hammer mill, to obtain a powder material for a cosmetic according to Example 13. The contained amount of DG4ISA in the powder material for a cosmetic of Example 13 was 0.1 mass%.

### [Example 14]

Five-tenths of a gram of DG4ISA, which was to serve as a surface treatment agent, was added to and dispersed in 10 g of n-hexane to prepare a liquid mixture. Next, 99.5 g of titanium oxide as a powder for a cosmetic was stirred using a mixer. The liquid mixture was dropped to and mixed with the powder for a cosmetic for 10 minutes to prepare a mixture. The mixture was subjected to a heat treatment at 110°C for 6 hours, and the resultant dry powder was pulverized using a hammer mill, to obtain a powder material for a cosmetic according to Example 14. The contained amount of DG4ISA in the powder material for a cosmetic of Example 14 was 0.5 mass%.

### [Example 15]

A powder material for a cosmetic according to Example 15 was obtained in a manner similar to that of Example 14, except that the temperature of the heat treatment was changed to 50°C. The contained amount of DG4ISA in the powder material for a cosmetic of Example 15 was 0.5 mass%.

### [Example 16]

A powder material for a cosmetic according to Example 16 was obtained in a manner similar to that of Example 14, except that the temperature of the heat treatment was changed to 70°C. The contained amount of DG4ISA in the powder material for a cosmetic of Example 16 was 0.5 mass%.

### [Example 17]

A powder material for a cosmetic according to Example 17 was obtained in a manner similar to that of Example 14, except that the temperature of the heat treatment was changed to 150°C. The contained amount of DG4ISA in the powder material for a cosmetic of Example 17 was 0.5 mass%.

### [Example 18]

One gram of DG4ISA, which was to serve as a surface treatment agent, was added to and dispersed in 10 g of n-hexane to prepare a liquid mixture. Next, 99.0 g of titanium oxide as a powder for a cosmetic was stirred using a mixer. The liquid mixture was dropped to and mixed with the powder for a cosmetic for 10 minutes to prepare a mixture. The mixture was subjected to a heat treatment at 110°C for 6 hours, and the resultant dry powder was pulverized using a hammer mill, to obtain a powder material for a cosmetic according to Example 18. The contained amount of DG4ISA in the powder material for a cosmetic of Example 18 was 1.0 mass%.

### [Example 19]

Five grams of DG4ISA, which was to serve as a surface treatment agent, was added to and dispersed in 10 g of n-hexane to prepare a liquid mixture. Next, 95.0 g of titanium oxide as a powder for a cosmetic was stirred using a mixer. The liquid mixture was dropped to and mixed with the powder for a cosmetic for 10 minutes to prepare a mixture. The mixture was subjected to a heat treatment at 110°C for 6 hours, and the resultant dry powder was pulverized using a hammer mill, to obtain a powder material for a cosmetic according to Example 19. The contained amount of DG4ISA in the powder material for a cosmetic of Example 19 was 5.0 mass%.

### [Example 20]

Ten grams of DG4ISA, which was to serve as a surface treatment agent, was added to and dispersed in 10 g of n-hexane to prepare a liquid mixture. Next, 90.0 g of titanium oxide as a powder for a cosmetic was stirred using a mixer. The liquid mixture was dropped to and mixed with the powder for a cosmetic for 10 minutes to prepare a mixture. The mixture was subjected to a heat treatment at 110°C for 6 hours, and the resultant dry powder was pulverized using a hammer mill, to obtain a powder material for a cosmetic according to Example 20. The contained amount of DG4ISA in the powder material for a cosmetic of Example 20 was 10.0 mass%.

### [Example 21]

Eleven grams of DG4ISA, which was to serve as a surface treatment agent, was added to and dispersed in 10 g of n-hexane to prepare a liquid mixture. Next, 89.0 g of titanium oxide as a powder for a cosmetic was stirred using a mixer. The liquid mixture was dropped to and mixed with the powder for a cosmetic for 10 minutes to prepare a mixture. The mixture was subjected to a heat treatment at 110°C for 6 hours, and the resultant dry powder was pulverized using a hammer mill, to obtain a powder material for a cosmetic according to Example 21. The contained amount of DG4ISA in the powder material for a cosmetic of Example 21 was 11.0 mass%.

### [Comparative Example 1]

A powder material for a cosmetic according to Comparative Example 1 was obtained in a manner similar to that of Example 1, except that isopropyltriisostearoyl titanate, which has a red color and is conventionally used, was used as a surface treatment agent. The contained amount of isopropyltriisostearoyl titanate in the powder material for a cosmetic of Comparative Example 1 was 2.0 mass%.

### [Comparative Example 2]

A powder material for a cosmetic according to Comparative Example 2 was obtained in a manner similar to that of Example 1, except that polyhydroxystearic acid (PHSA) was used as a surface treatment agent. The contained amount of PHSA in the powder material for a cosmetic of Comparative Example 2 was 2.0 mass%.

### [Comparative Example 3]

A powder material for a cosmetic according to Comparative Example 3 was obtained in a manner similar to that of Comparative Example 1, except that iron oxide (a mixture of yellow, red, and black iron oxides) was used as a powder for a cosmetic. The powder material for a cosmetic of Comparative Example 3 was added to a W/O type liquid foundation described below, and was used only for assessing the stability of the cosmetic.

### [Comparative Example 4]

A powder material for a cosmetic according to Comparative Example 4 was obtained in a manner similar to that of Comparative Example 1, except that talc powder was used as a powder for a cosmetic. The powder material for a cosmetic of Comparative Example 4 was added to a W/O type liquid foundation described below, and was used only for assessing the stability of the cosmetic.

### [Comparative Example 5]

A powder material for a cosmetic according to Comparative Example 5 was obtained in a manner similar to that of Comparative Example 2, except that iron oxide (a mixture of yellow, red, and black iron oxides) was used as a powder for a cosmetic. The powder material for a cosmetic of Comparative Example 5 was added to a W/O type liquid foundation described below, and was used only for assessing the stability of the cosmetic.

### [Comparative Example 6]

A powder material for a cosmetic according to Comparative Example 6 was obtained in a manner similar to that of Comparative Example 2, except that talc powder was used as a powder for a cosmetic. The powder material for a cosmetic of Comparative Example 6 was added to a W/O type liquid foundation described below, and was used only for assessing the stability of the cosmetic.

### [Comparative Example 7]

A titanium oxide powder was used as a powder for a cosmetic. The titanium oxide powder was directly used as a powder material for a cosmetic according to Comparative Example 7 without being subjected to a surface treatment.

### <Oil Agent Dispersibility Assessment Test 1>

It is known that the viscosity of a dispersion in which a powder material for a cosmetic is dispersed in an oil agent decreases with an increase in the dispersibility of the powder material for a cosmetic in the oil agent. Therefore, in an oil agent dispersibility assessment test 1, the viscosities of dispersions in which the powder materials for a cosmetic of Examples 1, 2, 7, 8, 9, 10, 11, and 12, and Comparative Examples 1 and 2 were dispersed in an oil agent were measured using a B-type viscometer. Each dispersion was prepared by stirring and mixing 60 g of a powder material for a cosmetic and 40 g of an oil agent at 1000 rpm for 10 minutes. As the oil agent, glyceryl tri(caprylate/caprate), which is an ester oil agent, squalene, which is a hydrocarbon oil agent, and dimethicone oil, which is a silicone oil agent, were used.

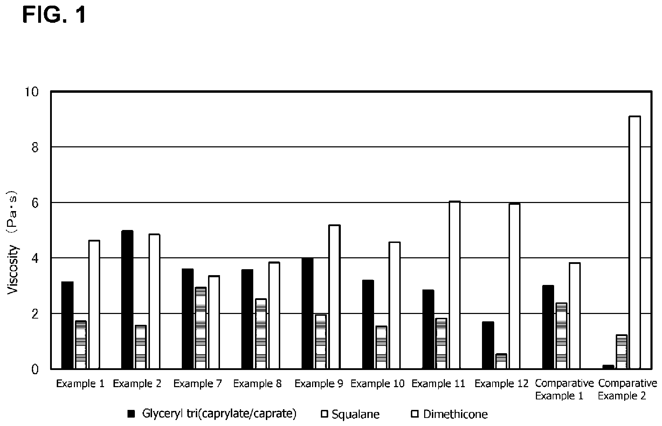

FIG. 1 is a graph showing the result of the oil agent dispersibility assessment test 1. The dispersions of the powder materials for a cosmetic of Examples 1, 2, 7, 8, 9, 10, 11, and 12 had a viscosity close to that of the dispersion of the powder material for a cosmetic of Comparative Example 1 in any of the oil agents, glyceryl tri(caprylate/caprate), squalane, and dimethicone oil. It was observed that the powder material for a cosmetic of the present invention had as excellent dispersibility in any of silicone oil agents, hydrocarbon oil agents, and ester oil agents as that of a powder material for a cosmetic surface-treated with isopropyltriisostearoyl titanate. It is considered that the powder material for a cosmetic of the present invention can be used as a substitute for a powder material for a cosmetic surface-treated with isopropyltriisostearoyl titanate, which has conventionally been used in many products. In particular, in the case where squalene is used as an oil agent, the dispersions of the powder materials for a cosmetic of Examples 1, 2, 9, 10, 11, and 12 had a viscosity lower than that of the powder material for a cosmetic of Comparative Example 1. In the case where dimethicone was used as an oil agent, the dispersion of the powder material for a cosmetic of Example 7 had a viscosity lower than that of the powder material for a cosmetic of Comparative Example 1. In this regard, it is considered that the powder material for a cosmetic of the present invention can be more suitably used in cosmetics employing a hydrocarbon oil agent than a powder material for a cosmetic surface-treated with isopropyltriisostearoyl titanate.

Meanwhile, it was observed that the powder material for a cosmetic of Comparative Example 2, in which PHSA was used as a surface treatment agent, had a high viscosity and does not have sufficient dispersibility in a dispersion employing dimethicone oil.

### <Dispersion Property Assessment>

In measurement of the oil absorption number of a powder in accordance with In JIS K 6217-4 "Determination of oil absorption number of carbon black," as an oil agent is gradually added to a sample which is being stirred by rotating blades, the mixture changes from a free-flowing powder to a viscous mass, and the torque on the rotating blade resulting from the change in viscous properties is recorded. The shape of the recorded torque curve is affected by the maximum amount of the oil agent absorbed by the powder, the wettability of the powder with respect to the oil agent, the hardness of the mass, and the like. Therefore, powders having similar torque curve shapes have similar properties with respect to an oil agent, i.e., similar dispersion properties. Given the above, the oil absorption numbers of the powder materials for a cosmetic of Example 1 and Comparative Examples 1 and 2 were measured using an absorptometer in accordance with JIS K 6217-4 "Determination of oil absorption number of carbon black," and the torque curve shapes obtained by the measurement were compared to assess the dispersion properties of the powders material for a cosmetic in an oil agent. Specifically, the torque curve was produced by placing 50 g of a powder material for a cosmetic in a mixing chamber, dropping an oil agent to the powder material for a cosmetic at a constant rate while the powder material for a cosmetic was being stirred by rotating blades in the mixing chamber, and at that time, measuring the torque applied to the rotating blade. As the oil agent, glyceryl tri(caprylate/caprate), which is an ester oil agent, an alkane having 9-12 carbon atoms, which is a hydrocarbon oil agent, and dimethicone oil, which is a silicone oil agent, were used.

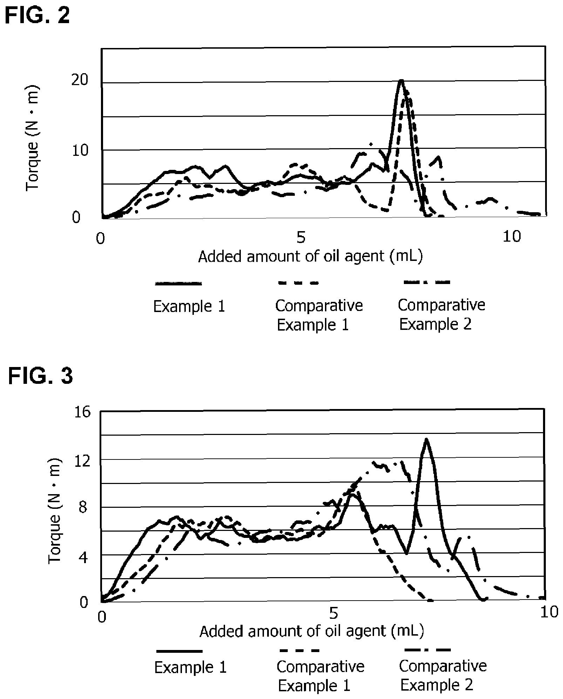

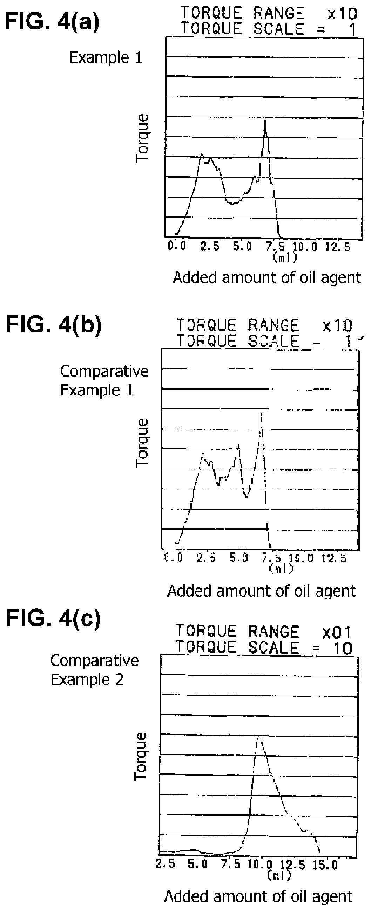

FIG. 2 is a torque curve obtained by determination of an oil absorption number using an ester oil agent. FIG. 3 is a torque curve obtained by determination of an oil absorption number using a hydrocarbon oil agent. FIG. 4 is a torque curve obtained by determination of an oil absorption number using a silicone oil agent. The powder material for a cosmetic of Example 1 had a torque curve shape similar to that of the powder material for a cosmetic of Comparative Example 1, whichever of a silicone oil agent, a hydrocarbon oil agent, and an ester oil agent was used. In particular, when an ester oil agent and a silicone oil agent were used, the torque curve shape of the powder material for a cosmetic of Example 1 was very similar to that of the powder material for a cosmetic of Comparative Example 1. It was demonstrated that the powder material for a cosmetic of the present invention has dispersion properties similar to those of a powder material for a cosmetic surface-treated with isopropyltriisostearoyl titanate, which has conventionally been used in many product, in silicone oil agents, hydrocarbon oil agents, and ester oil agents.

Meanwhile, when a silicone oil agent was used, the powder material for a cosmetic of Comparative Example 2, in which PHSA was used as a surface treatment agent, had a torque curve shape totally different from that of the powder material for a cosmetic of Comparative Example 1. Furthermore, the powder material for a cosmetic of Comparative Example 2 had a a very great maximum viscous drag torque that is about 10 times the maximum viscous drag torque of the powder material for a cosmetic of Comparative Example 1. Thus, it was observed that the powder material for a cosmetic of Comparative Example 2 had dispersion properties in a silicone oil agent that are significantly different from those of a powder material for a cosmetic surface-treated with isopropyltriisostearoyl titanate.

### <Oil Agent Dispersibility Assessment Test 2>

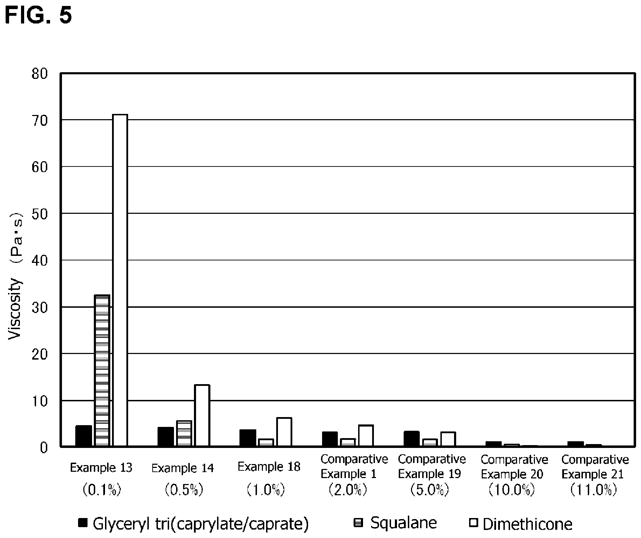

In general, in a surface treatment of a powder for a cosmetic, it is preferable that the final powder material for a cosmetic should have an appropriate amount of a surface treatment agent (ester compound). If the contained amount of the ester compound is too small, a sufficient effect is not likely to be obtained. If the contained amount of the ester compound is too great, the powder material for a cosmetic is likely to aggregate in a powder state after the surface treatment, which makes it difficult for the powder material for a cosmetic to be used in powder cosmetics (powder foundation, eye shadow, blush, etc.) in terms of texture, processing (the powder material for a cosmetic cannot be mixed with other colors), or the like. In this regard, in order to investigate the optimum contained amount of the ester compound in the powder material for a cosmetic, an oil agent dispersibility assessment test 2 was conducted on Examples 13, 14, 18, 19, 20, and 21, which had different DG4ISA concentrations, in a manner similar to that of the oil agent dispersibility assessment test 1. The results are shown in FIG. 5. Note that FIG. 5 also shows the result of Example 1 in the oil agent dispersibility assessment test 1 (2.0 mass%).

FIG. 5 is a graph showing the result of the oil agent dispersibility assessment test 2. Note that when the concentration of an untreated powder material for a cosmetic (a surface treatment agent was not added), such as that of Comparative Example 7, in a dispersion was similar to that which was used in the test (60 mass%), the powder material for a cosmetic was not able to be dispersed in glyceryl tri(caprylate/caprate), squalane, or dimethicone oil, so that paste was not obtained. Therefore, the viscosity of the untreated powder material for a cosmetic was not able to be measured. Meanwhile, powder materials for a cosmetic containing 0.1 mass% of the ester compound were able to form paste, and the viscosity was able to be measured. As a result, it was observed that the powder materials for a cosmetic containing 0.1 mass% of the ester compound had the effect of the surface treatment. It was also observed that the greater the contained amount of the ester compound, the lower the viscosity of the dispersion, and the better the dispersibility. Meanwhile, powder materials for a cosmetic containing at least 11 mass% of the ester compound had sufficient dispersibility in an oil agent, and aggregated in a powder state, which tended to make it difficult for the powder materials for a cosmetic to be used in powder cosmetics (powder foundation, eye shadow, blush, etc.) in terms of texture, processing (the powder material for a cosmetic cannot be mixed with other colors), or the like. Therefore, it was demonstrated that the contained amount of the ester compound in the powder material for a cosmetic is preferably 0.1-10%.

### <Water Repellency Test>

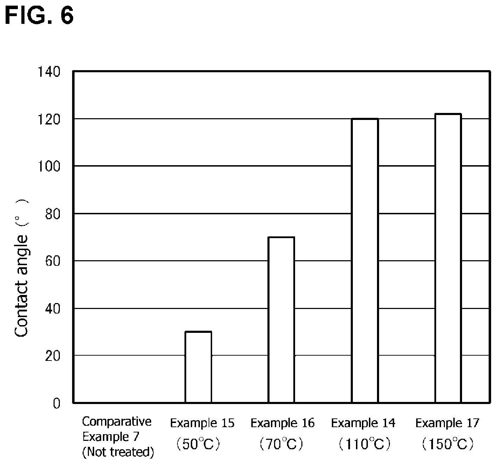

When a powder for a cosmetic is subjected to a surface treatment, a heat treatment step is a key step. If the treatment temperature in the heat treatment step is too low, sufficient water repellency and dispersibility are not likely to be obtained. If the treatment temperature is too high, the degradation, vaporization, and discoloration of the surface treatment agent are likely to occur. As described above, water repellency is represented by a contact angle. The contact angle is an angle formed by a tablet made of a powder material for a cosmetic and a dropped water droplet. The measured value of the contact angle indicates the degree of water repellency. Therefore, the greater the contact angle of a powder material for a cosmetic, the higher the water repellency thereof. In this regard, the contact angles of the powder materials for a cosmetic of Examples 14, 15, 16, and 17, which were subjected to a heat treatment at different treatment temperatures, and the untreated powder material for a cosmetic of Comparative Example 7, which was not subjected to a surface treatment, i.e., not to a heat treatment, were measured in a water repellency test as follows. Initially, a mold was filled with an appropriate amount of a powder for a cosmetic, which was then pressed with a compressive force of 10 MPa, to form a tablet for contact angle measurement. The tablet thus produced was placed in a contact angle measurement device (contact angle meter: LSE-B100, manufactured by NiCK Corporation). A water droplet was dropped to the tablet using a syringe. The contact angle (°) between the water droplet and the tablet was measured. The results are shown in FIG. 6.

FIG. 6 is a graph showing the result of the water repellency test. In Comparative Example 7 (not treated), the water droplet penetrates into the powder material for a cosmetic, and disappeared on the surface thereof, and therefore, there was no contact angle. As can be seen from FIG. 6, if the treatment temperature was lower than 70°C, sufficient water repellency tended not to be obtained. Meanwhile, if the treatment temperature was 70-150°C, sufficient water repellency was obtained. Note that if the treatment temperature was higher than 150°C, DG4ISA tended to vaporize and degrade. Therefore, it was demonstrated that the treatment temperature is preferably 70-150°C.

### <Cosmetic>

### [Examples 22 and 23 and Comparative Examples 8 and 9]

Cosmetics (W/O type liquid foundation) according to Examples 22 and 23 and Comparative Examples 8 and 9 were obtained using a production method described below. Note that the contained amount of each component is expressed in mass%.

### (Method for Producing W/O Type Liquid Foundation)

Initially, components B that are a powder component were mixed and stirred thoroughly using a mixer until a homogenous mixture was obtained. Next, components A that are an oil component were mixed, followed by heating to 80°C, and mixed and stirred thoroughly using a disper until a homogenous mixture was obtained. The mixture of the components B was gradually added to the mixture of the components A, which were kept stirred using the disper, while the resultant mixture was gradually cooled to 50°C. Components C that are an aqueous component were mixed together, followed by heating to 80°C, and homogenously dissolved, and thereafter, gradually cooled to 50°C. Finally, the mixture of the components C, which had been gradually cooled to 50°C, was gradually added to the mixture of the components A and B, which had been gradually cooled to 50°C, while the mixtures were being stirred using a disper, to obtain an emulsion. The emulsion was cooled to room temperature to obtain a W/O type liquid foundation.

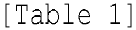

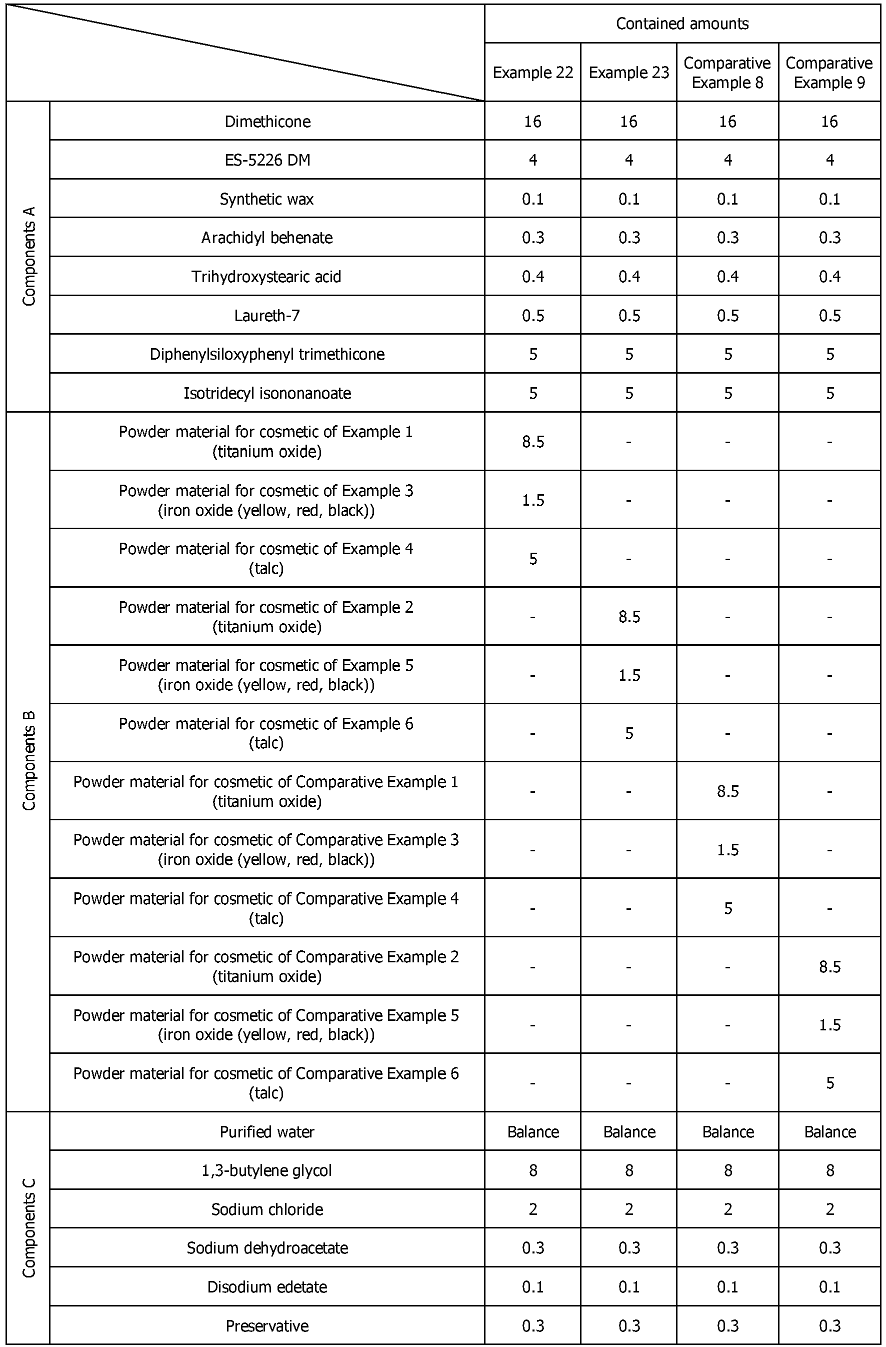

The cosmetics of Examples 22 and 23 and Comparative Examples 8 and 9 were each stored at room temperature and 45°C for 3 weeks. The stability of the cosmetics was assessed by visually observing the appearance of the cosmetics after storage.

The appearance of the cosmetics of Examples 22 and 23 was not altered after storage both at room temperature and 45°C. Thus, it was demonstrated that the cosmetic of the present invention has excellent stability.

Meanwhile, in the cosmetic of Comparative Example 8, red streaks occurred on the surface of the liquid foundation after storage both at room temperature and 45°C. This may be because the color of isopropyltriisostearoyl titanate itself used as a surface treatment agent appeared. In the cosmetic of Comparative Example 9, the color of the liquid foundation became denser after storage at 45°C, compared to the cosmetics of Examples 22 and 23 and Comparative Example 8. When the cosmetic of Comparative Example 9 was applied to the skin, the color was altered.

A sensory assessment test was conducted by a panel of 10 female assessors on each of the cosmetics of Examples 22 and 23 and Comparative Examples 8 and 9 in terms of feeling of use. In the sensory assessment test, each cosmetic was actually applied to the skin of the female assessors, and the female assessors describe the cosmetic by means of a questionnaire in terms of three aspects, "feeling of use," "color appearance," and "spreadability." In the questionnaire, each assessor answered each question on a rating scale of 1 to 5, and the average was calculated over all of the assessors. Assessment criteria are described below. As described below, the higher the value, the higher the rating. The results are shown in Table 2.
1: not acceptable for cosmetics
2: acceptable and not desirable for cosmetics
3: acceptable for cosmetics
4: desirable for cosmetics
5: very desirable for cosmetics

**[Table 2]**

| | Example 22 | Example 23 | Comparative Example 8 | Comparative Example 9 |
|---|---|---|---|---|
| Feeling of use | 4.8 | 4.6 | 4.6 | 3.8 |
| Cobr appearance | 4.8 | 4.5 | 3.8 | 3.5 |
| Spreadability | 4.6 | 4.2 | 4.2 | 3.2 |

As can be seen from Table 2, the cosmetics of Examples 22 and 23 had feeling of use, color appearance, and spreadability equivalent to or better than those of Comparative Example 8. The cosmetics of Examples 22 and 23 were very highly rated compared to the cosmetic of Comparative Example 9. Thus, the powder material for a cosmetic of the present invention is considered to be useful for make-up cosmetics.

### INDUSTRIAL APPLICABILITY

The powder material for a cosmetic of the present invention is applicable to cosmetics and the like, particularly, make-up cosmetics containing pigment, such as foundation, eye shadow, eyebrow liner, and blush.

## Claims

1. A powder material for a cosmetic comprising a powder for a cosmetic surface-treated with an ester compound of a fatty acid having 8-20 carbon atoms and glycerol.

2. The powder material for a cosmetic according to claim 1, wherein
the ester compound is at least one selected from the group consisting of diglyceryl tetraisostearate, diglyceryl triisostearate, glyceryl tri(caprylate/caprate), glyceryl dilaurate, glyceryl triisostearate, decaglyceryl monoisostearate, and decaglyceryl diisostearate.

3. The powder material for a cosmetic according to claim 1 or 2, wherein
the contained amount of the ester compound is 0.1-10 mass%.

4. The powder material for a cosmetic according to any one of claims 1-3, wherein
the powder for a cosmetic is at least one selected from the group consisting of titanium oxide, yellow iron oxide, red iron oxide, black iron oxide, talc, zinc oxide, silicon oxide, pearl mica, mica, and sericite.

5. A method for producing a powder material for a cosmetic, comprising:
mixing a powder for a cosmetic with a liquid mixture obtained by dispersing, suspending, or dissolving an ester compound of a fatty acid having 8-20 carbon atoms and glycerol in an organic solvent; and
heat-treating the mixture obtained in the mixing.

6. The method according to claim 5, wherein
in the heat-treating, a treatment temperature is set to 70-150°C.

7. The method according to claim 5 or 6, wherein
in the mixing, the liquid mixture is added to the powder for a cosmetic while the powder for a cosmetic is being stirred.

8. A cosmetic comprising the powder material for a cosmetic according to any one of claims 1-4.
